Europäisches Patentamt

European Patent Office

Office européen des brevets

(11) Publication number: **0 389 015**
**A2**

# EUROPEAN PATENT APPLICATION

(21) Application number: **90200438.1**

(22) Date of filing: **26.02.90**

(51) Int. Cl.⁵: **A61F  13/15**

(30) Priority: **20.03.89 US 325618**

(43) Date of publication of application:
**26.09.90 Bulletin  90/39**

(84) Designated Contracting States:
**AT BE CH DE DK ES FR GB GR IT LI LU NL SE**

(71) Applicant: **THE PROCTER & GAMBLE
COMPANY**
**One Procter & Gamble Plaza**
**Cincinnati Ohio 45202(US)**

(72) Inventor: **Furio, Diane Lynn**
**7225 Overcliff Rd.**
**Cincinnati, OH 45233(US)**
Inventor: **Johnson, Theresa Louise**
**11785 Rose Ln., Apt. C**
**Cincinnati, OH 45246(US)**
Inventor: **Benson, Douglas Herrin**
**Rt. 6, Box 203, Jamison Rd.**
**West Harrison, IN 47060(US)**

(74) Representative: **Suslic, Lydia et al**
**Procter & Gamble European Technical**
**Center N.V. Temselaan 100**
**B-1820 Strombeek-Bever(BE)**

(54) Absorbent structures with odor control material.

(57) Absorbent articles such as sanitary napkins, diapers, and the like, comprising a topsheet and an absorbent core, are provided with odor-controlling agents, especially molecular sieves, in the form of a comminuted sheet material. The sheet form simplifies the handling of the odor-controlling agents and the high-speed manufacture of the finished article. By comminuting the sheet, rather than using it in integral form, the sanitary and aesthetic advantages associated with modern topsheet materials are retained.

EP 0 389 015 A2

Xerox Copy Centre

# ABSORBENT STRUCTURES WITH ODOR CONTROL MATERIAL

## TECHNICAL FIELD

The present invention relates to absorbent structures, such as catamenials, diapers, bandages, adult incontinence garments, and the like, which comprise a fibrous or filamentous topsheet and an odor-control material.

## BACKGROUND OF THE INVENTION

A wide variety of absorbent structures designed not only to be efficient for the absorption of body fluids such as blood, urine, menses, and the like, but also to be sanitary and comfortable in-use are known in the literature. Disposable products of this type generally comprise some sort of fluid-permeable topsheet material, an absorbent core, and a fluid-impermeable backsheet material. Various shapes, sizes and thicknesses of such articles have been explored in an attempt to make their use more comfortable and convenient.

One of the greatest improvements in the comfort and convenience of such sanitary products has been in the development of new topsheet materials to replace the old style cotton gauze topsheets. In general, such topsheet materials are designed to provide quick passage of the discharged fluids through the topsheet and into the absorbent core. Certain topsheets are designed to prevent rewet caused by passage of fluid back through the topsheet, and this adds considerably to consumer comfort. Moreover, by selecting topsheet materials which do not, themselves, absorb fluids, a cleaner, more comfortable and more sanitary surface can be presented to the discharge point of the body fluid.

Another aspect of sanitary products which has been under investigation for many years is that of odor control. Many body fluids have an unpleasant odor, or develop such odors when in contact with air and/or bacteria for prolonged periods. The literature is replete with references relating to odor control in products such as diapers and catamenials.

Various odor-controlling agents have been disclosed in the literature, and many of these are designed either to be sprayed onto an absorbent core in diapers and catamenial products, or to be "dusted" thereon in the form of finely-divided particles. However, dusting or spraying (e.g., from an aqueous solution) of odor-controlling agents directly onto absorbent cores is a notion that does not take into account modern high speed manufacturing equipment and materials. For example, it would be difficult to spray an aqueous solution of an antibacterial onto air-laid absorbent diaper or catamenial cores moving at a speed of 500-600 cores per minute, and then to dry the cores sufficiently to allow them to be formed into a finished article. Using dry powders, especially finely-divided powders, is also problematic, inasmuch as the powders tend to be blown or vacuumed up from the cores by the processing equipment, especially on high speed lines.

Accordingly, it would be desirable to provide an odor-controlling agent in a form which would be amenable to fabrication into absorbent structures without the aforementioned manufacturing difficulties. One method would be to combine the odor-controlling agent into a separate sheet of material and place that sheet under the topsheet so that the discharged fluid quickly comes in contact with the sheet containing the odor-controlling agent immediately after passage through the topsheet.

However, it has been determined that this apparently simple solution to the problem raises some difficulties, particularly when employed with the newer "nonstaining" topsheet materials. For one thing, use of the odor-controlling agent in the form of a continuous sheet of material undesirably adds to the stiffness of the overall product. This is particularly true in regard to modern catamenial products, which are designed to be as soft, thin, dry, pliable and comfortable as possible. Moreover, placement of a continuous sheet of material containing the odor-controlling agent immediately under the nonstain topsheet undesirably gives the appearance (if not the fact) of stain being present on the topsheet, itself. This can be particularly disturbing to users who have come to value the sanitary appearance afforded by such topsheet materials.

Moreover, placing said continuous sheet of material between the topsheet and the absorbent core can undesirably affect the acquisition of fluid by the core.

The present invention provides a means for overcoming these difficulties by providing the odor-controlling agent in the form of discontinuous pieces, strips, or the like, rather than as a single continuous sheet underlying a nonstain topsheet. Accordingly, the present invention provides not only a simplified

2

means for manufacturing absorbent structures having odor-control properties without the difficulties associated with dustiness and drying, and fluid acquisition, noted hereinabove, but also retains the pleasant aesthetic properties afforded by the topsheet. These and other advantages associated with the present invention will be seen from the disclosure, hereinafter.

## BACKGROUND ART

The patent literature contains a considerable number of references relating to odor control in sanitary products such as diapers, bandages and catamenials. The following are illustrative.

U.S. 4,385,632 (5/31/83) by S. O. Odelhög assigned to Landstingens Inköpscentral teaches copper odor control agents used on the surface of absorbent articles.

U.S. 3,804,094 (4/16/74) by K. Dossou, M. Gascon, G. Manoussos, assigned to L'Oreal Fr teaches a periodic acid odor control agent used on the surface of an absorbent article.

U.S. 4,525,410 (6/25/85) by 2. Hagiwara, H. Ohki, S. Hoshino, S. Nohara, S. Ida, K. Tagawa, assigned to Kanebo, Ltd. and Kanto Chemical Co., Inc. teaches zeolite particles (doped with bactericidal cations) assertedly stably held in a fibrous web by incorporating some portion of meltable fibers in the web, and applying heat; said to be useful as the "outside cover layer" in, e.g., "general sanitary goods".

Jananese J63224734-A (88.09.19) Priority 87JP-058738 (87.03.16) J63224734 ASK KK relates to a paper comprising a powder or fiber obtained by grinding sepiolite, said paper having deodorizing capacity.

Japanese J63242261-A (88.10.07) 87JP-076111 J63242261 ASK KK relates to an odor-absorbing mat with sepiolite powder, a nonwoven fabric layer, and what appears to be a sheet to which the sepiolite is attached by adhesive.

U.S. 2,690,415 (9/28/54) by F. A. Shuler teaches particles of odor-absorbing materials uniformly affixed at the interstices of a permeable web by adhesive to provide an odor absorbent medium for, e.g., catamenials. Particulate carbon, silica gel and activated alumina are noted. Shifting/displacement of the particulates is assertedly avoided and the sheet is flexible.

U.S. 3,093,546 (6/11/63) by R. L. Atkinson, teaches halogenated diphenyl methane derivatives "advantageously placed on the surface of a catamenial dressing" to "obtain prompt deodorizing activity".

Jananese J54141857 (J87019865) teaches the manufacture of powder (including zeolites) sheets by laminating the powder between a first and second sheet. Powders include activated carbon, zeolite, etc. The abstract indicates use in catamenials or deodorizing materials.

BE-815446 (Abstract) teaches sanitary towels with chlorophyll crystals or activated carbon, either in the absorbent layer, on the surface, or (per abstract) between.

ABSCENTS (odor-control molecular sieve from Union Carbide) -Use in diapers and catamenials is specifically noted in Union Carbide brochure (A. J. Gioffre 1988). The brochure indicates that UC's market research shows potential benefits in such products.

Patents relating to various topsheets, diaper and catamenial designs, and the like, are listed in the Detailed Description and Examples, hereinafter. All patent documents cited in this specification are incorporated herein by reference.

## SUMMARY OF THE INVENTION

The present invention relates to absorbent structures, comprising:

(a) a flexible, fluid-receiving front face which comprises a sheet of nonabsorbent fibrous or filamentous web or network material, said sheet being fluid-permeable by virtue of a multiplicity of openings or channels passing therethrough;

.  (b) a fluid-retaining absorbent core beneath said front face (a) comprising a major portion of fluid-retaining material, and a minor portion of odor-control material which comprises multiple pieces of comminuted (e.g., diced or shredded) sheet material containing an odor-controlling agent;

(c) a backing sheet beneath said core (b) e.g., comprising a flexible, fluid-impermeable polymeric film or flushable/biodegradable sheet; and

(d) optionally, means for retaining said structure in an appropriate position to perform its absorbency function.

In one embodiment, the odor-controlling agent used herein is an antibacterial compound. In another embodiment, the odor-controlling agent is a water-insoluble particulate odor-absorbing material, or said particulate material in combination with an antibacterial compound. Such particulate odor-controlling agents include, for example, members selected from the group consisting of carbon, silica gel, zeolite (or, "molecular sieve"), activated alumina, and mixtures thereof.

In one preferred embodiment, the structures herein have a front face sheet which comprises a formaminous net (as described more fully, hereinafter). Sanitary products made from such structures include adult incontinence garments, absorbent pads (e.g., bed pads), pantiliners, catamenials and diapers, and also include bandages, foot pads, and the like.

In another preferred embodiment, the structures herein have a front face sheet which comprises a net of ribbon-like polymer filaments (as described more fully hereinafter). Sanitary products of the same type noted above can be prepared from such absorbent structures, including adult incontinence garments, absorbent pads, pantiliners, diapers and, most particularly, catamenials (sanitary napkins).

All percentages herein are by weight, unless otherwise specified.

## DETAILED DESCRIPTION

The articles disclosed herein can be prepared using constituents that are very well-known in current commercial practice, and reference can be made to the various patents mentioned herein and to the general sanitary products patent literature and trade catalogues for such items. Likewise, methods and apparatus for assembling disposable diapers, catamenials, bandages, and the like are known from patents and engineering literature.

While the constituents used in the assembly of catamenials, disposable diapers, and the like, are well-known, the following may be mentioned solely by way of example. It is to be understood that the present invention resides in the novel assemblage of such items, or their equivalents, into the odor-controlling absorbent structures disclosed herein, rather than in the constituents *per se*.

I. Front-face Material - The front-face (or, "topsheet") material used herein is, typically, a polymeric hydrophobic, fluid-permeable sheet. Hydrophobic sheet materials of the type typically employed in the practice of this invention can be prepared by methods well-described in the patent literature. For example, according to the process of U.S. Patent 4,636,419, Madsen et al, January 13, 1987, sheets comprising a network of ribboned filaments of two dissimilar chemical types, and with two dissimilar melting or softening points, are contacted and cooled to allow the formation of a network sheet characterized by said different transverse and longitudinal polymer materials. Such sheets can be used in the practice of this invention.

Another sheet material useful herein is the formaminous net comprising a reticular network of polymeric filaments, said net comprising two arrays of filaments oriented at a displacement angle of 20-90 degrees. Reference can be made to European Patent Application 0215417, filed 06.09.86, Sneyd et al, to further assist visualization of this sheet. The aforesaid sheet materials can be prepared using hydrophobic plastics such as polyethylene, polypropylene, PVC, and the like, and are well-known for use in absorbent products such as catamenials, and the like. Such sheet materials typically have a basis weight of 0.5-5.0 ounces/(yd$^2$ (0.0016 g/cm$^2$ - 0.016 g/cm$^2$), a caliper of 5-25 mils, an open area of 30-80% and a mesh of 20-40.

Such topsheet materials are generally noted for their stain resistance. The most preferred way to check stain appearance is by *in vivo* testing with users, using visual standards and standard statistical techniques. If desired, an *in vitro* test can be devised. An *in vitro* test protocol (which is only by way of illustration) is found in U.S. Patent 4,324,246, Mullane and Smith, April 13, 1982.

II. Absorbent Material - The absorbent material used herein can comprise any of the well-known fluid (e.g., blood-, urine- or menses-) absorbent materials such as cotton cloth, cellulose fibers, "super absorbent" polymers, and mixtures thereof.

As is well-known from recent commercial practice, fluid-retaining cores comprising absorbent gelling materials (sometimes referred to as "super-sorbers") are becoming broadly accepted by consumers. Such materials form hydrogels on contact with water (e.g., with urine, blood, and the like). One highly preferred type of hydrogel-forming, absorbent gelling material is based on polyacids, especially polyacrylic acid. Hydrogel-forming polymeric materials of this type are those which, upon contact with fluids (i.e., liquids) such as water or body fluids, imbibe such fluids and thereby form hydrogels. In this manner, fluid discharged into the absorbent structures herein can be acquired and held. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers

4

may comprise the entire gelling agent or may be grafted onto other types of polymer moieties such as starch or cellulose. Acrylic acid grafted starch materials are of this latter type. Thus the preferred absorbent gelling materials include hydrolyzed acrylonitrile grafted starch, acrylic acid grafted starch, polyacrylates, maleic anhydride-based copolymers and combinations thereof. Especially preferred absorbent gelling materials are the polyacrylates and acrylic acid grafted starch.

Whatever the nature of the polymer components of the preferred absorbent gelling materials, such materials will in general be slightly cross-linked. Crosslinking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer material; and (4) polyvalent metal compounds which can form ionic cross-linkages. Cross-linking agents of the foregoing types are described in greater detail in Masuda et al; U.S. Patent 4,076,663; Issued February 28, 1978. Preferred cross-linking agents are the di- or polyesters of unsaturated mono-or polycarboxylic acids with polyols, the bisacrylamides and the di-or triallyl amines. Especially preferred cross-linking agents are N,N'-methylenebisacrylamide, trimethylol propane triacrylate and triallyl amine. The cross-linking agent will generally comprise from about 0.001 mole percent to 5 mole percent of the preferred materials. More preferably, the cross-linking agent will comprise from about 0.01 mole percent to 3 mole percent of the absorbent gelling materials used herein.

The preferred, slightly cross-linked, hydrogel-forming absorbent gelling materials will generally be employed in their partially neutralized form. For purposes described herein, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization." Typically, commercial absorbent gelling materials have a degree of neutralization somewhat less than 90%.

The preferred absorbent gelling materials used herein are those which have a relatively high capacity for imbibing fluids encountered in the absorbent articles; this capacity can be quantified by referencing the "gel volume" of said absorbent gelling materials. Gel volume can be defined in terms of the amount of synthetic urine absorbed by any given absorbent gelling agent buffer and is specified as grams of synthetic urine per gram of gelling agent.

Gel volume in synthetic urine (see Brandt, et al, below) can be determined by forming a suspension of about 0.1-0.2 parts of dried absorbent gelling material to be tested with about 20 parts of synthetic urine. This suspension is maintained at ambient temperature under gentle stirring for about 1 hour so that swelling equilibrium is attained. The gel volume (grams of synthetic urine per gram of absorbent gelling material) is then calculated from the weight fraction of the gelling agent in the suspension and the ratio of the liquid volume excluded from the formed hydrogel to the total volume of the suspension. The preferred absorbent gelling materials useful in this invention will have a gel volume of from about 20 to 70 grams, more preferably from about 30 to 60 grams, of synthetic urine per gram of absorbent gelling material.

Another feature of the preferred absorbent gelling materials relates to the level of extractable polymer material present in said materials. Extractable polymer levels can be determined by contacting a sample of preferred absorbent gelling material with a synthetic urine solution for the substantial period of time (e.g., at least 16 hours) which is needed to reach extraction equilibrium, by then filtering the formed hydrogel from the supernatant liquid, and finally by then determining the polymer content of the filtrate. The particular procedure used to determine extractable polymer content of the preferred absorbent gelling agent buffers herein is set forth in Brandt, Goldman and Inglin; U.S. Patent 4,654,039; Issued March 31, 1987, Reissue 32,649. The absorbent gelling materials which are especially useful in the absorbent articles herein are those which have an equilibrium extractables content in synthetic urine of no more than about 17%, preferably no more than about 10% by weight of the absorbent gelling material.

The absorbent gelling materials hereinbefore described can be incorporated into the cores of the absorbent articles of the present invention in the form of discrete particles. Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

The size of the absorbent gelling material particles may vary over a wide range. For reasons of industrial hygiene, average particle sizes smaller than about 30 microns are less desirable. Particles having

5

a smallest dimension larger than about 2 mm may also cause a feeling of grittiness in the absorbent article, which is undesirable from a consumer aesthetics standpoint. Furthermore, rate of fluid absorption can be affected by particle size. Larger particles have very much reduced rates of absorption. Preferred for use herein are absorbent gelling material particles substantially all of which have a particle size of from about 30 microns to about 2 mm. "Particle Size" as used herein means the weighted average of the smallest dimension of the individual particles.

The amount of absorbent gelling material particles used in absorbent cores will depend upon the degree of absorbent capacity desired, and will generally comprise from about 2% to 50% by weight of the absorbent core, more typically from about 5% to 20% by weight of the absorbent core.

When absorbent gelling material particles are to be used in the cores of the absorbent articles herein, such cores can be prepared by any process or technique which provides a web comprising a combination of the fibers and the gelling material particles. For example, web cores can be formed by air-laying a substantially dry mixture of hydrophilic fibers and absorbent gelling material particles and, if desired or necessary, by densifying the resulting web. Such a procedure is described more fully in Weisman and Goldman; U.S. Patent 4,610,678; Issued September 9, 1986. As indicated in this U.S. Patent 4,610,678, the air-laid webs formed by such a procedure will preferably comprise substantially unbonded fibers and will preferably have a moisture content of 10% or less.

The density of the absorbent cores which comprise webs of hydrophilic fibers and absorbent gelling material particles can be of importance in determining the absorbent properties of the cores and of the absorbent articles in which such cores are employed. The density of such absorbent cores herein will preferably be in the range of from about 0.06 to about 0.3 g/cm$^3$, and more preferably within the range of from about 0.09 to about 0.22 g/cm$^3$. Typically the basis weight of the absorbent cores herein can range from about 0.02 to 0.12 g/cm$^2$.

Density values for cores of this type can be calculated from basis weight and caliper. Caliper is measured under a confining pressure of 0.137 psi (0.94 kPa). Density and basis weight values include the weight of the absorbent gelling materials and the odor-control material. Density of the cores herein need not be uniform throughout the core. Within the density ranges hereinbefore set forth, the cores can contain regions or zones of relatively higher or relatively lower density.

III. Backing Sheet - The backing sheet is conventional, and can comprise a fluid-impervious polymer sheet, for example polyethylene or polypropylene, that is thin enough to be flexible. A polyethylene sheet 0.001-0.5 mm thick is typical. Flushable or biodegradable backing sheets can also be used, e.g., with pantiliner devices herein.

IV. Odor-Controlling Agent - The odor-controlling agent employed in the practice of this invention can be a water-soluble antibacterial compound. Such materials include, for example, halogenated phenylene compounds (U.S. Patent 3,093,546), periodic acid (U.S. Patent 3,804,094), various copper compounds, especially copper acetate (U.S. Patent 4,385,632), various quaternary ammonium salts, which are well-known for their antibacterial properties, e.g., cetyl pyridinium chloride, and the like. Alternatively, antibacterial compounds can be used conjointly with various particulate materials which, in use in the presence of moisture, release the antibacterial agent. Zeolite materials, such as zeolites which are bactericidal by virtue of having absorbed therein and thereon various bactericidal cations such as copper, silver and zinc, can be advantageously used in the practice of this invention (U.S. Patent 4,525,410). In a preferred mode, the odor-controlling agent is a water-insoluble particulate odor-absorbing material such as chlorophyll particles, activated carbon granules (8-140 mesh), ion exchange resin (Japanese 87019865), activated alumina, and absorbent zeolite materials, including the well-known "molecular sieve" zeolites of the type A and X zeolites (generally in the 1-10 micron particle size range) and the zeolite materials marketed under the trade name ABSCENTS by the Union Carbide Corporation and UOP, and which are typically available as a white powder in the 3-5 micron particle size range (see: ABSCENTS, A New Approach for Odor Control by A. J. Gioffre, copyright 1988 by the Union Carbide Corporation).

The use of preferred zeolite (or, "molecular sieves") of the ABSCENTS type to control odors is fully described in U.S. Patent 4,795,482, January 3, 1989, to Gioffre and Marcus. In general, these preferred molecular sieve odor-controlling agents appear to function by entrapping odoriferous substances within their molecular lattice structures. Whatever their mode of action, these odor-controlling agents can be characterized by their physical parameters, as follows. These agents are reported by Gioffre and Marcus to be crystalline siliceous molecular sieves in which at least about 90, and preferably at least about 95, percent of the framework tetrahedral oxide units are SiO$_2$ tetrahedra and which have a sorptive capacity for water at 25° C and 4.6 torr of less than 10 weight percent. In the case of aluminosilicate molecular sieves, those preferred in the practice of this invention have a framework SiO$_2$/Al$_2$O$_3$ molar ratio of from about 35 to infinity, and preferably from 200 to 500. Such siliceous molecular sieves have a pore diameter of at least 5.5 Angstroms, preferably at least 6.2 Angstrons. Preferably the adsorption capacity for water vapor at 25°C and a water vapor pressure (p/p₀) of 4.6 torr is less than 6 weight percent. The efficacy of these preferred molecular sieves employed in the practice of the present invention is not dependent upon the presence of the water of hydration in the internal cavities of the microporous structure as a result of their hydrothermal formation. In fact, at least a major proportion, usually substantially all, of this original water of hydration is removed in the process of removing any pore-blocking templating agent which may be present in the adsorbent. Calcination effectively removes any organic moieties. Also, water washing or washing with a caustic or dilute mineral acid solution is advantageously utilized to remove extraneous synthesis reactants from the pore system. Lowering of the alkali metal content, particularly the nonzeolitic, i.e., occluded alkali metal compounds can also be beneficial. These procedures also serve to remove the original water of hydration.

As further disclosed by Gioffre and Marcus, such siliceous molecular sieves include the microporous crystalline aluminosilicates, i.e., the zeolitic molecular sieves as well as the so-called silica polymorphs. With respect to the latter compositions, their crystal lattices are ideally formed entirely of $SiO_2$ tetrahedral units, but the as-synthesized forms commonly contain at least trace amounts of aluminum derived from aluminum impurities in the synthesis reagents. The aluminosilicate molecular sieves comprise the large class of well-known crystalline zeolites. These high-silica molecular sieves are either commercially available or are prepared by methods well-known in the art, involving direct hydrothermal synthesis or involving certain types of crystal lattice dealuminations. A comprehensive review article by E. M. Flanigen concerning both "high" Si/Al zeolites and silica molecular sieves is published in "Proc. 5th Int. Conf. Zeolites, Naples, 1980", L. V. C. Rees, ed., Heyden, London, pp. 760-780.

With respect to the foregoing odor-controlling agents, it is important that their pore system be open so that the internal cavities of the crystals be accessible to the odor molecules. In the case of the aluminosilicates or silica polymorphs produced using large organic templating ions such as tetraalkylammonium ions, it is necessary to remove charge balancing organic ions and any occluded templating material in order to permit adsorption of the odor molecules. In such a removal process and also in the removal of inorganic debris, the original water of hydration is also removed. Upon exposure to the atmosphere, a portion of the water of hydration is reacquired, but this does not affect the characteristics of the molecular sieves which are preferred for the practice of the present invention, i.e., the molecular sieves can be employed in either a hydrated or dehydrated state, but, in general, the dehydrated state is preferred. In the case of most of the dealumination procedures referred to above, the original water of dehydration is also removed, and can similarly be replaced, if desired, for the practice of the invention.

More specifically, Gioffre and Marcus disclose that the class of medium to large pore siliceous molecular sieves, from which the original, as-synthesized water of hydration has been substantially removed, and which have a capacity for adsorbed water of not greater than 10, and preferably not greater than 6, weight percent when measured at 25°C and a water vapor pressure (p/p₀) of 4.6 torr, function in an extraordinary manner with respect to odor elimination. Many of the synthetic zeolites prepared using organic templating agents are readily prepared in a highly siliceous form - some even from reaction mixtures which have no intentionally added aluminum. These zeolites are markedly organophilic and include ZSM-5 (U.S. Patent 3,702,886); ZSM-11 (U.S. Patent 3,709,979); ZSM-35 (U.S. Patent 4,016,245); ZSM-23 (U.S. Patent 4,076,842); and ZSM-38 (U.S. Patent 4,046,859) to name only a few. It has been found that the silica molecular sieves known as silicalite and F-silicalite are particularly suitable for use in the present invention and are thus preferred. These materials are disclosed in U.S. Patents 4,061,724 and 4,073,865, respectively. To the extent the aforesaid siliceous sieves are synthesized to have $SiO_2/Al_2O_3$ ratios greater than 35, they are frequently suitable for use in the present articles without any additional treatment to increase their degree of hydrophobicity. Molecular sieves which cannot be directly synthesized to have both sufficiently high Si/Al and/or degree of hydrophobicity ratios can be subjected to dealumination techniques, fluorine treatments and the like, which result in organophilic zeolite products. High-temperature steaming procedures for treating zeolite Y which result in hydrophobic product forms are reported by P. K. Maher et al, "Molecular Sieve Zeolites", Advan. Chem. Ser. 101, American Chemical Society, Washington, D.C., 1971, p. 266. A more recently reported procedure applicable to zeolite species generally, involves dealumination and the substitution of silicon into the dealuminated lattice site. This process is disclosed in U.S. Patent 4,503,023 issued March 5, 1985 to Skeels et al. Halogen or halide compound treatments for zeolites to increase their hydrophobicity are disclosed in U.S. Patents 4,569,833 and 4,297,335. Steam-treated zeolite Y, prepared per U.S. Patent 4,331,694, and denominated "LZ-10", is a particularly useful odor-controlling agent.

Various modified zeolite-type materials, such as the manganese-aluminum-phosphorus-silicon-oxide molecular sieves described in U.S. Patent 4,793,833, Lok et al, assigned to UOP, can be used herein. See also U.S. Patents 4,604,110; 4,437,429; and 4,648,977, for other zeolitic odor-controlling compositions.

Optional Retaining Means - The absorbent structures herein can optionally, but preferably, be provided with means to hold them in place on or near the user's body to allow the structures to perform their intended function. For example, diapers and incontinence garments can be provided with well-known commercially-available tape fasteners. Sanitary napkins can be provided with glue stripes facing outward on their backsheet in well-known fashion. Various pins, clips and fasteners of well-known types can optionally be employed.

Odor-Control Material - It is to be understood that the odor-control material employed in the practice of this invention is not, simply, the odor-controlling agent, *per se*, added to the absorbent structure. Rather, the odor-controlling agent is formed into strips, particles, or the like. In a typical method, this forming process involves combining the odor-controlling agent with a suitable carrier material, forming said agent/carrier mixture into a sheet, and then cutting, slicing or otherwise comminuting said sheet into the desired size range. The preparation of an odor-control material in this fashion is illustrated in Example I. Standard papermaking equipment is used.


EXAMPLE 1


Southern Softwood Kraft (SSK) wood pulp is introduced into a papermaking apparatus (Formar, Noble & Wood Company) and passed onto the forming screen of the apparatus. While in the damp state on the forming screen, a 10% (wt.) aqueous suspension of zeolite-type molecular sieve powder (ABSCENTS; Union Carbide Corporation; 3-5 micron average particle size) is sprayed onto the damp pulp at a rate sufficient to give approximately 60% wt. (range 50-62%) ABSCENTS loading on/in the resulting sheet (dry weight basis). The pulp/ABSCENTS mix is thereafter dried (165-179 °C) to provide a sheet/ABSCENTS composite which has, generally, the appearance and consistency of commercial paper toweling (caliper ca. 12 mils; basis weight range 42-55 lbs/3000 ft$^2$) (ca. 19-25 kg/280 m$^2$).

The sheet prepared in the foregoing manner is thereafter comminuted to provide confetti-like pieces 3/32 in. x 5/8 in. (ca. 0.238 cm x 1.59 cm) by passing the above-prepared sheet through a commercial paper shredding machine (Ideal Paper Shredder - Trade Mark DESTROYIT, 4001 Cross-Cut). The resulting pieces are a preferred odor-control material employed in the practice of the present invention.

The "confetti" pieces of odor-control material are then introduced into a fluid-retaining absorbent core which comprises a major portion of fluid-retaining material and a minor proportion of said odor-control material. 0.8 g of ABSCENTS-containing odor-control material (60% loading of ABSCENTS) prepared in the foregoing manner is airlaid onto and into the surface and uppermost layers of an airlaid absorbent core comprising 8.5-9.5 grams of fluid-retaining NSK air-felt. The core is formed as a pad (surface area 18.14 in$^2$; 117 cm$^2$) which is suitable for use in a sanitary napkin.


EXAMPLE II


A disposable baby diaper using the odor-control material of Example I is prepared as follows. The dimensions listed are for a diaper intended for use with a child in the 6 to 10 kilogram size range. These dimensions can be modified proportionately for different size children, or for adult incontinence briefs, according to standard practice.

1. Backsheet: 0.025-0.070 mm polyethylene; width at top and bottom 33 cm; notched inwardly on both sides to a width-at-center of 28.5 cm; length 50.2 cm.

2. Topsheet: per U.S. Patent 4,636,419, described hereinabove; width at top and bottom 33 cm; notched inwardly on both sides to a width-at-center of 28.5 cm; length 50.2 cm.

3. Absorbent core: air-laid wood pulp fibers, Taber stiffness range 7-9.5, 8.4 mm thick, calendered; width at top and bottom 28.6 cm; notched inwardly at both sides to a width-at-center of 10.2 cm; length 44.5 cm. Odor-control material of Example I (0.8 g) air-laid uniformly, but discontinuously, onto the fluid-receiving surface of core.

4. Elastic leg bands: four individual rubber strips (2 per side); width 4.77 mm; length 370 mm; thickness 0.178 mm (all the foregoing dimensions being in the relaxed state).

The diaper of Example II is prepared in standard fashion by positioning the core-plus-odor control material covered with the topsheet on the backsheet and gluing.

The elastic bands (designated "inner" and "outer", corresponding to the bands closest to, and farthest from, the core, respectively) are stretched to ca. 50.2 cm and positioned between the topsheet/backsheet along each longitudinal side (2 bands per side) of the core. The inner bands along each side are positioned ca. 55 mm from the narrowest width of the core (measured from the inner edge of the elastic band). This provides a spacing element along each side of the diaper comprising the flexible topsheet/backsheet material between the inner elastic and the curved edge of the core. The inner bands are glued down along their length in the stretched state. The outer bands are positioned ca 13 mm from the inner bands, and are glued down along their length in the stretched state. Since the topsheet/backsheet assembly is flexible, the glued-down bands contract to elasticize the sides of the diaper.

## EXAMPLE III

A sanitary napkin is prepared as follows. The absorbent core containing the "confetti" as prepared in Example I is calendered to a caliper of 1 mm. On top of this core is placed a cover comprising a topsheet made from the formaminous net (EPO 0215417, cited above) having a caliper of 10 mils and an open area of 55%. The thus-covered core is placed on a fluid-impermeable, flexible (0.03 mm) polyethylene backing sheet, and the structure is bonded together as a unitary antille by spot gluing.

## EXAMPLE IV

Following the procedure of Example I, confetti-like (1.9 cm x 0.6 cm) pieces of odor-control material comprising odor-controlling agents loaded on paper are prepared, as follows.

| Odor-Controlling Agent | Loading (% wt.) |
|---|---|
| (A) Cupric Acetate | 2% |
| (B) Copper-Zeolite A (1-10 microns) | 40% |
| (C) Zinc-Zeolite A (1-10 microns) | 35% |
| (D) Periodic Acid | 0.5% |
| (E) Silica Gel | 30% |
| (F) Alumina (1-5 micron) | 20% |
| (G) Activated Carbon | 12% |

The above-described odor-controlling materials are formed into sanitary napkins in the manner of Example III.

## EXAMPLE V

A highly absorbent, thin pad for use in sanitary napkins, disposable diapers, and the like, is prepared by dry-mixing the following components.

| Component | Wt. Ratio in Mix |
|---|---|
| Fibers* | 85 |
| Absorbent Gelling Material** | 15 |
| Odor-Control Material*** | 2 |

*Southern softwood slash pine.
**SANWET IM 1000; Sanyo Co., Japan; acrylate grafted starch, average particle size 250 micron range.
***Confetti pieces, per Example I. 60% ABSCENTS loading.

Absorbent pads are prepared by air-laying the above mixture. Pad basis weight 390 g/m². A hydraulic press can be used to compress the pads to a thickness, typically in the 1-5 mm range. The pad is bonded to a front face material and backsheet material, in the manner of Example III, to provide sanitary napkins, diapers, adult incontinence articles, bed pads, and the like.

## EXAMPLE VI

The pad of Example V is prepared, but with the SANWET being replaced by "low-extractables" polyacrylate absorbent gelling material, as disclosed by Brandt et al, U.S. Reissue Patent 32,649, cited hereinabove. The front face material is the network sheet of U.S. Patent 4,636,419 and the backsheet is flexible polyethylene. ABSCENTS loading 1.4 g.

## EXAMPLE VII

A pantiliner suitable for use between menstrual periods, and which can be disposed of in a toilet (i.e., "flushable") comprises a pad according to Example V, interposed between the topsheet of U.S. Patent 4,636,419 and a fibrous, nonwoven, flushable backsheet.

## EXAMPLE VIII

The absorbent core of Example I is modified by adding 500 ppm cetyl pyridinium chloride (CPC) to the SSK pulp (spray-on). In use as a sanitary napkin, the CPC provides odor control benefits in conjunction with the ABSCENTS odor-controlling agent.

**Claims**

1. An absorbent structure, comprising:
(a) a flexible, fluid-receiving front face which comprises a sheet of nonabsorbent fibrous or filamentous network material, said sheet being fluid-permeable by virtue of a multiplicity of openings or channels passing therethrough;
(b) a fluid-retaining absorbent core beneath said front face (a), comprising a major portion of fluid-retaining material and a minor portion of odor-control material;
(c) a backing sheet beneath said core (b); and
(d) optionally, means for retaining said structure in an appropriate position to perform its absorbency function;
said structure being characterized in that said odor-control material comprises multiple pieces of comminuted sheet material containing an odor-controlling agent.
2. A structure according to Claim 1, wherein the odor-controlling agent is an antibacterial compound.

3. A structure according to Claim 1 wherein the odor-controlling agent is a water-insoluble particulate odor-absorbing material, or said particulate material in combination with an antibacterial compound.

4. A structure according to Claim 3 wherein the odor-controlling agent is a member selected from the group consisting of carbon, silica gel, zeolite, siliceous molecular sieve, activated alumina, and mixtures thereof.

5. An adult incontinence garment, absorbent pad, pantiliner, catamenial or diaper according to Claim 4.